# EUROPEAN PATENT APPLICATION

(11) **EP 1 749 509 A1**
(43) Date of publication of application: **07.02.2007**
(21) Application number: 05017149.5
(22) Date of filing: 05.08.2005
(51) Int. Cl.: A61F 13/15, A61L 15/22, A61L 15/60

(54) **Process for making a liquid absorbing thermoplastic material**

(71) Applicant: The Procter and Gamble Company, Cincinnati, Ohio 45202 (US)
(72) Inventor: Gonzales, Denis, 1150 Bruxelles (BE); Lunetto, Pietro, 65025 Manoppello (Pescara) (IT); Pompei, Enzo, 65132 Pescara (IT); Toro, Carlo, 65012 Cepagatti (Pescara) (IT)
(74) Representative: Veronese, Pancrazio

(57) **Abstract**

The present invention relates to an improved process for making liquid absorbing thermoplastic materials comprising a thermoplastic polymeric composition and an absorbent material in particle form dispersed in the thermoplastic polymeric composition. Such liquid absorbing thermoplastic material can be utilised in the absorbent cores of disposable absorbent articles, such as sanitary napkins, panty liners, interlabial devices, tampons, disposable diapers, incontinence pads, wound dressings, nursing pads and the like for completely or partially substituting said absorbent cores.

## Description

### Field of the Invention

The present invention relates to a process for making a liquid absorbing thermoplastic material, comprising a thermoplastic polymeric composition having absorbent particles dispersed therein. Such liquid absorbing thermoplastic materials can be utilized in a number of end uses where liquid absorption is desired, for example in the absorbent core of disposable absorbent articles, such as sanitary napkins, panty liners, interlabial devices, tampons, disposable diapers, incontinence pads, wound dressings, nursing pads and the like for completely or partially substituting said absorbent core.

### Background of the Invention

In general the absorption and retention of aqueous liquids, particularly body fluids such as urine, menses, etc., are accomplished by use of absorbent articles containing absorbent materials. Such articles include sanitary napkins, panty liners, interlabial devices, tampons, disposable diapers, incontinence pads, wound dressings, nursing pads, and the like. Generally, the most used absorbent materials are cellulose materials (preferably, defiberised wood pulp) and superabsorbent materials. In particular, when referring to disposable diapers or sanitary napkins and the like presently available in the market, the cellulose materials are in the form of bat or sheet, typically further containing particulate absorbent materials, usually referred to in the art as superabsorbents or hydrogelling materials, which allow to manufacture thin but very absorbent core structures. A primary need in incorporating superabsorbent material in particle form within an absorbent structure is its stabilization, in order to counteract the tendency of powdered material to bunch up or agglomerate, hence providing an uneven absorptive capacity in the absorbent structure, or also to dust off the structure itself. Known approaches are for example to adhesively fix the particles into a fibrous structure, or to disperse the powdered superabsorbent material in a fibrous matrix, e.g. cellulose pulp, and fix it in place mechanically e.g. by calendaring or embossing. An alternative approach is to blend a superabsorbent particulate material into a thermoplastic matrix, e.g. a thermoplastic composition. The superabsorbent containing thermoplastic composition can be typically extruded or coated in any desired position and pattern onto a suitable substrate, to be then incorporated into an absorbent article, thus entirely providing the absorbent material in the article, or alternatively integrating a more traditional fibrous absorbent structure, with no risk of dust off of the particulate material, or displacement within the absorbent structure of the article. For example, EP 1013291 and WO 98/27559 describe a hot melt adhesive containing a superabsorbent polymer. WO 99/57201 illustrates compositions comprising a thermoplastic component and a superabsorbent polymer, said compositions in form of a film layer or applied to a disposable absorbent article with various hot melt adhesive application techniques. Our copending applications WO 03/049777 and WO 04/028427 respectively describe preferred thermoplastic compositions comprising a matrix of a thermoplastic polymeric composition and superabsorbent particles dispersed therein, which have a particularly effective fluid acquisition and handling capacity, and absorbent articles comprising superabsorbent containing thermoplastic compositions arranged in a pattern of unattached spaced apart zones.

The technology of composite thermoplastic materials comprising a matrix of a thermoplastic composition and particles of superabsorbent material dispersed therein has provided a solution to the problem of powdered superabsorbent material "instability" within absorbent structures in absorbent articles, preventing particle displacement as e.g. dust-off, agglomeration, or bunching up, and safeguarding the end users of absorbent articles virtually from any undesired contact with the superabsorbent particles upon normal use. This technology has also allowed the use in absorbent articles of superabsorbent materials in a much smaller particle size than previously adopted for traditionally manufactured fibrous absorbent structures with superabsorbent materials. Such reduced average particle size, generally in the range below 150µ, preferably from 10µ to 40µ, as disclosed for example in WO 04/028427, is advantageous not only in terms of better liquid handling and absorption capacity of the corresponding thermoplastic absorbent compositions, due to the increased surface/volume ratio provided by smaller particles, but also in an easier processability of the thermoplastic material, which may have lower viscosities at the process conditions, typically a hot melt extrusion onto a substrate.

However, powdered superabsorbent materials also pose health risks to those involved in the manufacturing process, particularly when they are manufactured in very small particle sizes as mentioned above. The finely powdered superabsorbent material can become airborne and can be inhaled by workers. Once inhaled, the superabsorbent material absorbs liquid within the respiratory passages swelling to many time its original size. This can result in blocked air passages and potentially traumatic health complications.

According to the known technique, liquid absorbing thermoplastic materials comprising a matrix of a thermoplastic composition with superabsorbent particles dispersed therein are manufactured by providing a) the superabsorbent material in the desired, and possibly very small, particle size, usually achieved by grinding coarser particles, b) the components of the thermoplastic composition, and c) by uniformly mixing them all upon sufficient heating, in order to melt the components of the thermoplastic composition. Hence the superabsorbent material has to be typically handled in a very small particle size, namely manufactured, shipped and then processed in the production line of the liquid absorbing thermoplastic composition. Shipping of superabsorbent materials in very low particle size is also subjected to severe limitations in many countries, based on health and environmental grounds. On the other hand provision of superabsorbent material in the desired very low particle size starting from coarser and environmentally less demanding particles directly in the same line of production of the final liquid absorbing thermoplastic material is not always convenient, since it necessarily implies addition of a grinding station to the production line, which does not allow much flexibility in the manufacturing process of the liquid absorbing thermoplastic material, and increases complexity. It also implies in any case handling of fine superabsorbent particles at a certain stage of the process.

Hence there is the need for a process for making a liquid absorbing thermoplastic material comprising a matrix of a thermoplastic composition and absorbent particles, typically particles of superabsorbent material, dispersed therein which is more flexible and allows the use of particles in the desired low particle size, without the limitations and drawbacks created by the handling of such very fine particles.

It is therefore an object of the present invention to provide an improved process for making a liquid absorbent thermoplastic material, which allows a simpler and safer handling of the absorbent materials in very low particle sizes, virtually with no limitation due to health and environmental issues related to finer particles.

Particularly, it is an object of the present invention to provide a process for making a typically liquid absorbing thermoplastic material wherein particles of superabsorbent material can be manufactured in whichever low particle size, and then possibly further handled, e.g. stocked or shipped, before provision to the liquid absorbing thermoplastic material production line.

### Summary of the Invention

The present invention provides a process for making a liquid absorbing thermoplastic material comprising a matrix of a thermoplastic polymeric composition and particles of absorbent material dispersed in the matrix, wherein the thermoplastic polymeric composition comprises at least two components. The process comprises the following steps:
providing the particles of absorbent material,
mixing the particles of absorbent material with a first component in the liquid state of the at least two components forming a pre-mix,
mixing the pre-mix with a second component of the thermoplastic polymeric composition in the molten state.

### Brief Description of the Drawings

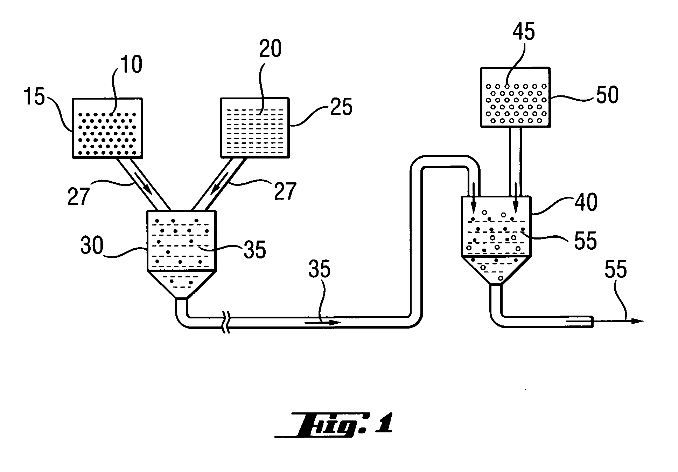

Figure 1 shows a schematic view of a production line for the manufacture of a liquid absorbing thermoplastic material according to the present invention.

### Detailed Description of the Invention

By "liquid" as herein used is meant water based fluids or liquids such as urine, menses, serum, blood, sweat, mucous as well as other aqueous solutions generally defined as body fluids, but it is not intended to exclude other water based fluids.

By "room temperature" as herein used is conventionally meant a temperature of 25°C, as known in the art.

For purposes of the present invention, viscosity has to be meant as melt viscosity at a certain temperature, which is determined with any suitable apparatus as known in the art. Particularly, viscosity can be determined with the procedure and apparatus described on page 22 of the already cited PCT application WO 99/57201, to which reference is made, in the paragraph headed "Melt Viscosity", possibly with the necessary adjustments, e.g. spindle selection, for measuring viscosities below 10 centipoises.

For purposes of the present invention, particle size is defined as the dimension of a particle which is determined by means of any suitable method known in the art for particle sizes comprised in the range according to the present invention. Particularly indicated are laser light scattering analysis or laser diffraction analysis. The average particle size of a given sample is defined as the particle size corresponding to a cumulative distribution of 50% of the particles of the sample. In other words, the average particle size of a given sample of absorbent material particles is defined as the particle size which divides the sample in half on a mass basis, i.e., half of the sample by weight will have a particle size greater than the average particle size and half of the sample by weight will have a particle size less than the particle size.

The process of the present invention will be herein described with reference to the production of a liquid absorbing thermoplastic material wherein the particulate absorbent material is in fact a water-insoluble water-swellable material in particle form, but of course the present invention refers to any liquid absorbing thermoplastic material comprising a different absorbent particulate material.

Figure 1 shows a production line for the manufacture of a liquid absorbing thermoplastic material comprising a matrix of a thermoplastic polymeric composition and particles of water-insoluble water-swellable absorbent material dispersed therein. The matrix of thermoplastic polymeric composition comprises at least two components.

The particles of water-insoluble water-swellable absorbent material 10 are provided from a supply means, for example a container 15, and the first component 20 in the liquid state of the thermoplastic polymeric composition is provided from a respective suitable supply means, typically a tank or vessel 25. The particles 10 and the first component 20 in the liquid state are conveyed in the desired amounts and proportions by suitable means, e.g. the tubing 27, to a mixing device 30, where they are uniformly mixed to form a pre-mix 35. Preferably, the first component 20 of the thermoplastic polymeric composition is liquid at room temperature (25°C), such that it can be directly provided in the liquid state from the tank or vessel 25 to the mixing device 30. The pre-mix 35 is such a case is typically a slurry which is more or less viscous or pasty at room temperature. Alternatively, in a less preferred embodiment of the process of the present invention, the first component 20 can be solid or semi-solid at room temperature (25°C), and brought to the liquid state by suitably heating it with known heating means before or upon providing it to the mixing device 35. In a particularly preferred embodiment of the present invention, the first component 20 is selected such that at room temperature it is liquid, and also has a viscosity below 30.000 centipoise, preferably below 10.000 centipoise, more preferably below 5.000 centipoise, even more preferably between 1 and 4.000 centipoise, most preferably between 10 and 3.000 centipoise. Low viscosity at room temperature of the liquid first component 20 provides the advantage of an easier and more effective mixing with the particles of water-insoluble water-swellable absorbent material 10 in order to form the pre-mix 35 in the mixing device 30. Less energy is in fact required for the mixing step, as well as for handling and conveying the first component 20, typically from the tank or vessel 25 to the mixing device 35, and of the pre-mix 35 as well further of the mixing device 35. A uniform mixing of the particles of water-insoluble water-swellable absorbent material 10 and of the first component 20 is also more easily and effectively achieved owing to the preferred relatively low viscosity of the first component 20 at room temperature.

If desired, a controlled heating can be also provided to a first component which is liquid already at room temperature, in order to further reduce its viscosity and preferably bring it in the desired range for the mixing step where the pre-mix is formed. Said preferred viscosities can be also provided by suitably heating and melting a first component in said mixing step in the less preferred embodiment when said first component is solid at room temperature.

The pre-mix 35 is then further processed in order to form the final liquid absorbing thermoplastic material. This further process can be performed directly in the same production line, right after the formation of the pre-mix 35, or alternatively the pre-mix can be temporarily stored, in order to be subsequently fed to the production line of the liquid absorbing thermoplastic material, in the same facility, or alternatively shipped to another facility for this final production step. In any case, the handling, storing and/or shipping of the pre-mix 35 constituted by the first component 20 preferably liquid at room temperature, comprising the particles of water-insoluble water-swellable absorbent material 10 dispersed therein, is greatly simplified with respect to the handling, storing and/or shipping of the particles of water-insoluble water-swellable absorbent material alone.

In order to provide the liquid absorbing thermoplastic material the pre-mix 35 is conveyed by a suitable means, not illustrated in Figure 1, e.g. via a pump, to a melting and mixing station 40 where a second component 45 meant to constitute the thermoplastic polymeric composition is also fed in the desired amount from a container 50. Alternatively, multiple other components can be also provided in the desired amounts, for example from respective containers, to the melting and mixing station 40, where they are suitably heated and melted, with known means and according to the known technology of thermoplastic polymeric composition production, and compounded with the pre-mix 35 containing the first component 20 of the thermoplastic polymeric composition, with the particles of water-insoluble water-swellable absorbent material 10 already dispersed therein. The final liquid absorbing thermoplastic material 55 comprising the matrix of the thermoplastic polymeric composition with the particles of water-insoluble water-swellable absorbent material 10 dispersed therein is then formed, and can be provided for further uses and production steps according to known techniques, for example directly applied in the molten state onto a suitable substrate, or solidified and stored in suitable forms, e.g. pellets, for further uses. The final liquid absorbing thermoplastic material 55 is typically solid at room temperature.

The pre-mixing of the particles of water-insoluble water-swellable absorbent material 10 with a first component 20 in liquid form of the thermoplastic polymeric composition in the process of the present invention, solves the problems generally implied by the handling of materials in form of fine and very fine particles, particularly the environmental and health problems related to the handling of particles of water-insoluble water-swellable absorbent material. This in turn allows the use of particles having a very low average size, which are generally advantageous in that they allow an easier processability of the thermoplastic material comprising the particulate material. As it is known, addition of solid particles into a matrix of a thermoplastic polymeric composition increases the overall viscosity of the thermoplastic material in the molten state at given conditions, when compared with the viscosity of the same matrix without particles, at the same conditions. Selection of particles having a preferred low or very low average size reduces this effect, providing a less viscous thermoplastic material in the molten state at the same conditions, hence facilitating the handling and processing of the thermoplastic material, for example when applying it onto a substrate by means of known extrusion or hot melt coating techniques. Smaller particles also provide an increased surface to volume ratio compared to larger particles, hence generally enhancing their functionality. Particularly, water-insoluble water-swellable absorbent material in particle form provides enhanced absorption capacity and absorption rate when in very small particle size, owing to the increased surface to volume ratio. It is particularly preferred that the absorbent particulate material comprised in the matrix of thermoplastic polymeric composition to form the liquid absorbing, thermoplastic material made with the process of the present invention has an average particle size below 150 µ, preferably below 40 µ, more preferably between 1 µ and 30 µ. Particularly preferred is an average particle size between 10 µ and 20 µ.

Particulate absorbent material of the preferred very low particle size can be handled and also shipped with no concern of health and environmental risks according to the process of the present invention, since it can be provided, e.g. by grinding coarser particles to the desired particle size, or directly synthesized in the desired particle size, and then mixed with the first component in the liquid state of the matrix of thermoplastic polymeric composition to form the pre-mix, and then actually handled and/or shipped as a semi-liquid slurry, with no exposure of dust or fine particles. The pre-mix moreover constitutes an ingredient of the final liquid absorbing thermoplastic material, which can be prepared by simply compounding the pre-mix with the other component or components of the thermoplastic matrix by suitably melting and mixing according to known technique, with no need of separating the particulate material from the first component in the liquid state before the final compounding step, as it would happen with liquid carriers for particulate materials which are known in the art.

According to the preferred embodiment described so far, the liquid absorbing thermoplastic material prepared according to the process of the present invention comprises a matrix of a thermoplastic polymeric composition with particles of a water-insoluble water-swellable absorbent material dispersed therein.

In general, particulate absorbent materials for the manufacturing of a liquid absorbing thermoplastic material according to the process of the present invention can be selected among known particulate water-insoluble, water-swellable absorbent materials, as already mentioned, or also among particulate liquid gelling materials.

Particulate water-insoluble water-swellable absorbent materials comprise known, typically crosslinked, absorbent materials which are usually referred to as "hydrogels", "super absorbents", "absorbent gelling materials" (AGM). Such materials, upon contact with aqueous fluids, especially aqueous body fluids, imbibe such fluids and thus form hydrogels by swelling of their own three-dimensional network provided by crosslinking. These absorbent materials are typically capable of absorbing large quantities of aqueous body fluids, and are further capable of retaining such absorbed fluids under moderate pressures. These absorbent materials are typically in the form of discrete, non fibrous particles, even if super absorbents in fibre form are known.

Any commercially available super absorbent material in particle form is suitable for the liquid absorbing thermoplastic material made with the process of the present invention. Suitable super absorbent materials for use herein will most often comprise a substantially water-insoluble, slightly crosslinked, partially or fully neutralized, polymeric gelling material. This material forms a hydrogel upon contact with water. Such polymer materials can be prepared from polymerizable, unsaturated, acid-containing monomers. Preferred materials are polyacrylate based superabsorbent polymers in particle form.

Particulate absorbent materials to be included in the liquid absorbing thermoplastic material made with the process of the present invention can also comprise particulate liquid gelling materials, which are materials, typically not crosslinked, which upon contact with liquid form a gel creating a three-dimensional network by interacting with the molecules present in the liquid, such as typically proteins, lipids, and so on in body fluids. Liquid gelling materials can be selected for example among polysaccharides, starches, modified cellulose. Preferred liquid gelling materials according to the present invention are cationic polysaccharides, among which particularly preferred are chitosan and its derivatives, where the creation of a three-dimensional network upon contact with liquid, and hence gelification, is achieved by formation of electrostatic bonds between the positively charged cationic groups of the cationic polysaccharide and the negatively charged electrolytes contained in the fluid.

Suitable chitosan materials to be used herein include substantially water-soluble chitosan. Suitable chitosan materials for use herein may generally have a wide range of average molecular weights, typically ranging from 1.000 to 10.000.000, preferably from 2.000 to 1.000.000. Particularly suitable chitosan materials for use herein are chitosan salts, particularly water-soluble chitosan salts. A variety of acids can be used for forming chitosan salts, namely inorganic and organic acids. Chitosan salts formed by the reaction of chitosan with an amino acid are also suitable for use herein.

Examples of chitosan salts formed with an inorganic acid include, but are not limited to, chitosan hydrochloride, chitosan hydrobromide, chitosan phosphate, chitosan sulphonate, chitosan chlorosulphonate, chitosan chloroacetate and mixtures thereof. Examples of chitosan salts formed with an organic acid include, but are not limited to, chitosan formate, chitosan acetate, chitosan lactate, chitosan glycolate, chitosan malonate, chitosan epoxysuccinate, chitosan benzoate, chitosan adipate, chitosan citrate, chitosan salicylate, chitosan propionate, chitosan nitrilotriacetate, chitosan itaconate, chitosan hydroxyacetate, chitosan butyrate, chitosan isobutyrate, chitosan acrylate and mixtures thereof. It is also suitable to form a chitosan salt using a mixture of acids including, for example, both inorganic and organic acids. A particularly preferred chitosan salt for use herein is chitosan lactate.

The absorbent particulate material is preferably provided in the preferred low average particle size as specified above.

Preferably, according to the process of the present invention, the pre-mix comprises from 40% to 80%, preferably from 50% to 70%, by weight of the pre-mix, of particles of absorbent material, and from 20% to 60%, preferably from 30% to 50%, by weight of said pre-mix, of said first component of said thermoplastic polymeric composition. Preferably, the final liquid absorbing thermoplastic material made with the process of the present invention comprises from 10% to 90%, preferably from 15% to 70%, more preferably from 20% to 60%, by weight of the liquid absorbing thermoplastic material, of particles of absorbent material.

The average particle size of the liquid absorbent particulate material, e.g. of a water-insoluble water-swellable absorbent material, used herein is preferably low, typically below 150 µ, preferably below 40 µ, more preferably between 1 µ and 30 µ. An average particle size between 10 µ and 20 µ is particularly preferred for water-insoluble water-swellable absorbent materials.

Small particle sizes are preferred herein as this results in optimum performance and processability for the liquid absorbing thermoplastic composition prepared according to the process of the present invention. In an embodiment of the present invention, particulate absorbent materials comprised in the liquid absorbing thermoplastic material made with the process of the present invention can be synthesized according to known processes such as they have the desired low average particle size. Examples are superabsorbent materials having substantially a spherical shape commercially available from Sumitomo Seika in different average particle sizes under the trade name Aquakeep® 10SH-NF. However, it is preferred that the desired low average particle size is achieved by suitably grinding a coarser material. Ground particulate absorbent materials, for example ground superabsorbent materials, are definitely cheaper compared to materials directly prepared in the selected average particle size, and are commonly used in absorbent articles. Manufacturing, such as for example by grinding, and generally handling particulate materials in low and very low average particle sizes, particularly superabsorbent materials, pose as already explained health and environmental risks, especially when said materials are manufactured in the low and extremely low average particle sizes preferred for the liquid absorbing thermoplastic material made with the process of the present invention. According to a preferred embodiment of the present invention, the process for making the liquid absorbing thermoplastic material described so far comprises the further step of grinding the particles of the absorbent material directly into the pre-mix with known means to the selected low average particle size, such that the particulate absorbent material can be provided to the pre-mixing step in a relatively large average particle size which is easily available in the market and also processed/handled without special care.

Alternatively, the grinding step can be performed onto to particulate absorbent material already dispersed and compounded into the thermoplastic polymeric composition, typically in the molten state, as a last step in order to obtain the particulate absorbent material in the selected low average particle size in the liquid absorbing thermoplastic material.

Although the above preferred process, namely in its alternative execution, includes a grinding station in the production line of the liquid absorbing thermoplastic material, it has the advantage that the particulate absorbent material in low and very low average particle size is never present as such, i.e. as a free, dry powder, in any manufacturing step either of the pre-mix or of the liquid absorbing thermoplastic material, thus completely avoiding the health and environmental issues related to the handling of particulate absorbent material in very fine average particle size.

It is preferable that the absorbent material in particle form, typically preferably the water-insoluble water-swellable absorbent material, is present in the liquid absorbing thermoplastic material made with the process of the present invention in an amount from 10% to 90%, more preferably from 15% to 70% and most preferably from 20% to 60% by weight of the total liquid absorbing thermoplastic material.

The liquid absorbing thermoplastic material made according to the process of the present invention further comprises as an essential element a matrix of a polymeric thermoplastic composition typically at a level from 10% to 90%, preferably from 30% to 85%, more preferably from 40% to 80% by weight of the liquid absorbing thermoplastic material.

Any thermoplastic polymeric composition known to the skilled person and suitable to form the matrix in the liquid absorbing thermoplastic material made by the process of the present invention can be used herein, provided it comprises at least two components, with a first component intended to be mixed in the liquid state with the absorbent particulate material to form the pre-mix. Preferably, the first component is inert to the particulate material, that is to say it is substantially non reactive with the absorbent material in particle form. Preferably, the absorbent material in particle form is also substantially insoluble in the first component, such that the absorbent material keeps its particulate state in the pre-mix with the first component and in the final liquid absorbing thermoplastic material. In the preferred embodiment where the first component of the thermoplastic polymeric composition is liquid at room temperature, preferably it has at room temperature a viscosity below 30.000 centipoise, more preferably below 10.000 centipoise, even more preferably below 5.000 centipoise, still more preferably between 1 and 4.000 centipoise, most preferably between 10 and 3.000 centipoise. The thermoplastic polymeric compositions for use herein typically comprise thermoplastic polymers as an essential element. Thermoplastic polymer or mixtures of polymers are present in amounts typically ranging from about 5% to 99%, preferably 10% to 90%, more preferably from 30% to 70%, most preferably from 40% to 60% with respect to the total weight of the thermoplastic polymeric composition forming the matrix.

The matrix of thermoplastic polymeric composition can be also formulated as a hot melt adhesive, as it is known in the art, hence typically comprising e.g. a thermoplastic polymer, a plasticiser and a tackifier resin. Of course the hot melt adhesive constituting the matrix must comprise at least a first component to be mixed in the liquid state with the particulate material, preferably a first component which is liquid at room temperature.

Liquid absorbing thermoplastic materials comprising particles of water-insoluble water-swellable material are already known in the art, and can be prepared according to the process of the present invention. For example, materials described in patent applications WO 98/27559 and WO 99/57201 can be prepared with the process of the present invention.

In a particularly preferred embodiment of the present invention, the thermoplastic polymeric composition comprises one or more thermoplastic polymers and a suitable compatible plasticiser, wherein the plasticiser is the first component which is mixed in the liquid state with the absorbent particulate material, preferably with particles of water-insoluble water-swellable material, in order to form the pre-mix. The pre-mix is subsequently compounded with the other thermoplastic polymer or polymers comprised in the thermoplastic polymeric composition.

Preferably the suitable compatible plasticiser is liquid at room temperature and even more preferably has the viscosity at room temperature as specified above.

According to a preferred embodiment of the present invention, the thermoplastic polymer can be selected from the group consisting of polyurethanes, poly-ether-amides block copolymers, polyethylene-acrylic acid and polyethylene-methacrylic acid copolymers, polyethylene oxide and its copolymers, ethylene acrylic esters and ethylene methacrylic esters copolymers, poly lactide and copolymers, polyamides, polyesters and copolyesters, polyester block copolymers, sulfonated polyesters, poly-ether-ester block copolymers, poly-ether-ester-amide block copolymers, polyacrylates, polyacrylic acids and derivatives, ionomers, polyethylene-vinyl acetate with a vinyl acetate content of at least 28% by weight, polyvinyl alcohol and its copolymers, polyvinyl ethers and their copolymers, poly-2-ethyl-oxazoline and derivatives, polyvinyl pyrrolidone and its copolymers, thermoplastic cellulose derivatives, poly-caprolactone and copolymers, poly glycolide, polyglycolic acid and copolymers, polylactic acid and copolymers, polyureas, and the suitable compatible plasticiser can be selected from the group consisting of citric acid esters, tartaric acid esters, glycerol and its esters, sucrose esters, adipates, sebacates, sorbitol, epoxidized vegetal oils, polymerised vegetal oils, polyols, phthalates, liquid polyesters, glycolates, glycols and polyglycols and their derivatives, sorbitan esters, phosphates, monocarboxylic fatty acids (C8-C22) and their derivatives, mineral oils, high boiling point alcohols, glycerin, glycol ethers, which are preferably liquid at room temperature (or 25°C).

Particularly preferred thermoplastic polymers are selected from thermoplastic poly-ether-amide block copolymers (e.g. Pebax™), thermoplastic poly-ether-ester-amide block copolymers, thermoplastic polyester block copolymers (e.g. Hytrel™), thermoplastic polyurethanes (e.g. Estane™), and polyethylene-vinyl acetate with a vinyl acetate content of at least 28% by weight.

Preferred plasticisers are mineral oils, high boiling point alcohols, glycerin, and glycol ethers.

Particularly preferred plasticisers are polyethylene glycols, polypropylene glycols, and derivatives thereof, suitably selected such as they are liquid and have the preferred viscosity at room temperature.

A particularly preferred liquid absorbing thermoplastic composition comprising particles of superabsorbent material, which can be made with the process of the present invention, is described in patent application WO 03/49777. This application discloses highly preferred thermoplastic polymeric base materials for use in the liquid absorbent thermoplastic compositions to be applied in the absorbent articles of the present invention, which have a water absorption capacity at least greater than 30%, preferably greater than 40%, more preferably greater than 60% and most preferably greater than 90%, when measured according to the Water Absorption Test described herein in accordance with ASTM D 570-81, on a film 200 µm thick. The intrinsic absorbency of the polymeric base material/matrix allows for a more effective diffusion of the body fluid within the matrix and, consequently, for a better spreading of the body fluid which can reach a greater number of absorbent material particles which in turn give rise to a better utilization of the absorbent material.

Highly preferred liquid absorbent thermoplastic compositions described in WO 03/49777 are those showing good integrity in wet state and hence having a tensile strength in wet state which is at least 20%, preferably at least 40%, and more preferably 60% of the tensile strength of said composition in dry state. Said tensile strengths are evaluated according to the Tensile Strength Test described herein. It should be appreciated that by selecting a thermoplastic base material, in the liquid absorbent thermoplastic composition herein having a higher value of water absorption, the absorbent composition will have better liquid absorption/handling characteristics, while not compromising on tensile strength in wet state. Indeed such absorbent composition will remain substantially intact and have sufficient tensile strength for its intended use, also upon liquid absorption.

### Example according to the invention

A liquid absorbing thermoplastic material is prepared according to the following process. A particulate superabsorbent material wherein 100% of the particles has an average particle size below 25 µ, as measured e.g. by Laser Light Scattering Method, is prepared by suitably grinding the material available from Nippon Shokubai Co. Ltd. under the trade name Aqualic CA Type L74 to the desired particle size e.g. in a Fluid Bed Counter Mill AFG from Hosokawa Alpine. A pre-mix is formed by adding and uniformly dispersing at room temperature the particulate superabsorbent material into a polyethylene glycol (M.W. 400) available from Dow Europe GmbH under the trade name Carbowax^{™} PEG 400E. The pre-mix has the following composition in percent by weight:
- 40%: PEG 400
- 60%: Aqualic L74

The liquid absorbing thermoplastic material is prepared by compounding a thermoplastic polyether-amide block copolymer available from Atofina (France) under the trade name Pebax MV 3000 in the molten state with the pre-mix and with Irganox B 225 (anti oxidant agent) available from Ciba-Geigy. The liquid absorbing thermoplastic material has the following composition:
- 24%: Pebax MV 3000
- 30%: PEG 400
- 45%: Aqualic L74
- 1%: Irganox B 225

## Claims

1. A process for making a liquid absorbing thermoplastic material comprising a matrix of a thermoplastic polymeric composition and particles of an absorbent material dispersed in said matrix, wherein said thermoplastic polymeric composition comprises at least two components, said process comprising the following steps:
providing said particles of absorbent material,
mixing said particles of absorbent material with a first component in the liquid state of said at least two components forming a pre-mix,
mixing said pre-mix with a second component of said thermoplastic polymeric composition in the molten state.

2. The process according to claim 1, wherein said process comprises the further step of grinding said particles of absorbent material into said pre-mix to a selected average particle size.

3. The process according to claim 1, wherein said process comprises the further step of grinding said particles of absorbent material into said thermoplastic polymeric composition to a selected average particle size.

4. The process according to any preceding claim, wherein said particles of absorbent material are particles of water-insoluble water-swellable absorbent material.

5. The process according to any of claims 1 to 3, wherein said particles of absorbent material are particles of liquid gelling material.

6. The process according to any preceding claim, wherein said first component of said thermoplastic polymeric composition in said pre-mix is liquid at room temperature.

7. The process according to claim 6, wherein said first component has a viscosity at room temperature below 30.000 centipoise, preferably below 10.000 centipoise, more preferably below 5.000 centipoise, even more preferably between 1 and 4.000 centipoise, most preferably between 10 and 3.000 centipoise.

8. The process according any preceding claim, wherein said particles of absorbent material have an average particle size below 150 µ, preferably below 40 µ, more preferably between 1 µ and 30 µ.

9. The process according to any preceding claim, wherein said thermoplastic polymeric composition comprises a thermoplastic polymer and a suitable compatible plasticiser.

10. The process according to claim 9, wherein said first component in said pre-mix is said suitable compatible plasticiser.

11. The process according to claims 9 or 10, wherein said thermoplastic polymer is selected from the group consisting of polyurethanes, poly-ether-amides block copolymers, polyethylene-acrylic acid and polyethylene-methacrylic acid copolymers, polyethylene oxide and its copolymers, ethylene acrylic esters and ethylene methacrylic esters copolymers, poly lactide and copolymers, polyamides, polyesters and copolyesters, polyester block copolymers, sulfonated polyesters, poly-ether-ester block copolymers, poly-ether-ester-amide block copolymers, polyacrylates, polyacrylic acids and derivatives, ionomers, polyethylene-vinyl acetate with a vinyl acetate content of at least 28% by weight, polyvinyl alcohol and its copolymers, polyvinyl ethers and their copolymers, poly-2-ethyl-oxazoline and derivatives, polyvinyl pyrrolidone and its copolymers, thermoplastic cellulose derivatives, poly-caprolactone and copolymers, poly glycolide, polyglycolic acid and copolymers, polylactic acid and copolymers, polyureas,
and said suitable compatible plasticiser is selected from the group consisting of citric acid esters, tartaric acid esters, glycerol and its esters, sucrose esters, adipates, sebacates, sorbitol, epoxidized vegetal oils, polymerised vegetal oils, polyols, phthalates, liquid polyesters, glycolates, glycols and polyglycols and their derivatives, sorbitan esters, phosphates, monocarboxylic fatty acids (C₈-C₂₂) and their derivatives, mineral oils, high boiling point alcohols, glycerine, glycol ethers, which are preferably liquid at room temperature.

12. The process according to any preceding claim, wherein said pre-mix comprises from 40% to 80%, preferably from 50% to 70%, by weight of said pre-mix, of said particles of absorbent material, and from 20% to 60%, preferably from 30% to 50%, by weight of said pre-mix, of said first component of said thermoplastic polymeric composition.

13. The process according to any preceding claim, wherein said liquid absorbing thermoplastic material comprises from 10% to 90%, preferably from 15% to 70%, more preferably from 20% to 60%, by weight of said liquid absorbing thermoplastic material, of said particles of absorbent material.
